# EUROPÄISCHE PATENTSCHRIFT

(11) **EP 1 626 930 B1**
(45) Veröffentlichungstag und Bekanntmachung des Hinweises auf die Patenterteilung: **21.04.2010**
(21) Anmeldenummer: 04733323.2
(22) Anmeldetag: 17.05.2004
(51) Int. Cl.: C01B 31/02, C23C 18/02, C23C 18/12

(54) **VERFAHREN ZUR HERSTELLUNG VON POROESEM, KOHLENSTOFFBASIERTEM MATERIAL**
METHOD FOR PRODUCING A POROUS, CARBON-BASED MATERIAL
PROCEDE POUR PRODUIRE UN MATERIAU POREUX A BASE DE CARBONE

(30) Priorität: 16.05.2003 DE 10322182
(43) Veröffentlichungstag der Anmeldung: 22.02.2006
(73) Patentinhaber: CINVENTION AG, 65203 Wiesbaden (DE)
(72) Erfinder: BISCHOFSBERGER, Norman, 65183 Wiesbaden (DE); BAN, Andreas, 64285 Darmstadt (DE); MAYER, Bernhard, 55130 Mainz (DE); GOLDMANN, Dov, 65189 Wiesbaden (DE); RATHENOW, Jörg, 65203 Wiesbaden (DE); ASGARI, Soheil, 65203 Wiesbaden (DE)
(74) Vertreter: Hansen, Norbert
(86) Internationale Anmeldenummer: PCT/EP2004/005277
(87) Internationale Veröffentlichungsnummer: WO 2004/101433

(56) Entgegenhaltungen:
- WO-A-97/43473
- WO-A-99/52838
- DE-A- 10 051 910
- GB-A- 1 163 442
- GB-A- 1 513 235
- US-A- 4 318 948
- US-A- 5 209 979
- PATENT ABSTRACTS OF JAPAN Bd. 0122, Nr. 70 (C-515), 27. Juli 1988 (1988-07-27) & JP 63 050480 A (DENKI KAGAKU KOGYO KK), 3. März 1988 (1988-03-03)
- PATENT ABSTRACTS OF JAPAN Bd. 0176, Nr. 27 (C-1131), 19. November 1993 (1993-11-19) & JP 5 194056 A (OJI PAPER CO LTD), 3. August 1993 (1993-08-03) in der Anmeldung erwähnt
- PATENT ABSTRACTS OF JAPAN Bd. 0101, Nr. 61 (C-352), 10. Juni 1986 (1986-06-10) & JP 61 012918 A (OUJI SEISHI KK), 21. Januar 1986 (1986-01-21) in der Anmeldung erwähnt

## Beschreibung

Die vorliegende Erfindung betrifft ein Verfahren zur Herstellung von porösem, kohlenstoff-basiertem Material durch Karbonisierung von Polymerfolie in einer Atmosphäre, die im wesentlichen frei von Sauerstoff ist bei Temperaturen im Bereich von 80°C bis 3500°C.

Poröse, kohlenstoffbasierte Materialien finden im Bereich der Fluidtrennung seit längerem Anwendung. Derartige Materialien können als Adsorbentien, Membranschichten oder selbsttragende Membranen in geeigneter Form hergestellt und verwendet werden. Die vielfältigen Möglichkeiten sowohl die Porosität als auch die chemischen Eigenschaften kohlenstoffbasierter Materialien gezielt zu verändern, machen diese Materialien insbesondere für selektive Fluidtrennaufgaben besonders interessant.

Im Stand der Technik sind eine Reihe von Verfahren zur Herstellung von porösen kohlenstoffbasierten Materialien in flächiger Form, insbesondere in Blattform beschrieben. So wird beispielsweise in der WO 02/32558 ein Verfahren zur Herstellung von flexiblen und porösen Adsorbentien auf Basis von Kohlenstoff umfassenden Materialien beschrieben, wobei eine flächige Grundmatrix, deren Bestandteile im wesentlichen durch Wasserstoffbrückenbindungen zusammengehalten werden auf einer Papiermaschine hergestellt, und anschließend pyrolysiert wird. Die in dieser internationalen Anmeldung verwendeten Ausgangsmaterialien sind im wesentlichen Faserstoffe verschiedenster Art, da diese üblicherweise auf Papiermaschinen verwendet werden und die einzelnen Fasern im hergestellten Papier dann im wesentlichen durch Wasserstoffbrückenbindungen zusammengehalten werden.

Ähnliche Verfahren sind beispielsweise in der japanischen Patentanmeldung JP 5194056 A sowie der japanischen Patentanmeldung JP 61012918 beschrieben. In diesen Dokumenten sind ebenfalls Papierherstellungsprozesse beschrieben, mit denen aus organischen Fasern oder Kunststofffasern sowie Pulpe Papierblätter erzeugt werden, die mit Phenolharz behandelt und anschließend getrocknet, heißgepresst und in einer Inertgasatmosphäre karbonisiert werden. Auf diese Weise lassen sich dicke, poröse Kohlenstoffblätter mit Beständigkeit gegenüber Chemikalien und elektrischer Leitfähigkeit erhalten.

Nachteil der vorbeschriebenen Verfahren ist es jedoch, dass die verwendeten Fasermaterialien im Ausgangsmaterial in Abhängigkeit ihrer Faserstärke und Faserlänge, sowie deren Verteilung im flächenartigen Papiermaterial die Dichte und damit auch die Porosität des resultierenden Kohlenstoffmaterials nach der Pyrolyse weitgehend vorherbestimmen, so dass bei zu groß dimensionierten Poren weitere aufwändige Nachbearbeitungsschritte wie chemische Dampfphaseninfiltration notwendig sind, um durch Abscheidung weiteren Kohlenstoffmaterials die Poren zu verengen.

Darüber hinaus lassen sich nach den Verfahren des Standes der Technik nur Ausgangsmaterialien verwenden, die in einem notwendigerweise wässrigen Papierverarbeitungsprozess verwendbar sind, was die Auswahl der möglichen Ausgangsmaterialien insbesondere im Bereich der hydrophoben Kunststoffe stark einschränkt. Gerade derartige hydrophobe Kunststoffe, wie beispielsweise Polyolefine, sind jedoch aufgrund ihres hohen relativen Kohlenstoffgehalts und der einfachen Verfügbarkeit in gleichbleibender Qualität gegenüber Naturstofffasern oftmals bevorzugte Ausgangsmaterialien.

GB 860,342 beschreibt die Herstellung von Graphit aus regenerierter Cellulose bei einer Temperatur von 2500°C in Inertgas.

JP 2000-335909 beschreibt die Herstellung eines karbonisierten Films durch Karbonisierung eines porösen Films aus hochtemperaturbeständigem Polyimid in anaerober Atmosphäre.

JP 10-099 664 beschreibt die Herstellung eines karbonisierten Membran zur Gastrennung aus einem Cardo- Polymer durch Erhitzen in anaerober Atmosphäre.

Es besteht daher ein Bedürfnis nach einem kostengünstigen und einfachen Verfahren zur Herstellung poröser kohlenstoffbasierter Materialien, welches ohne die Notwendigkeit der Verwendung von aus Fasern hergestellten papierartigen Materialien auskommt.

Aufgabe der vorliegenden Erfindung ist es daher, ein Verfahren zur Herstellung poröser, im wesentlichen kohlenstoffbasierter Materialien zur Verfugung zu stellen, welches aus billigen und hinsichtlich ihrer Eigenschaften breit variablen Ausgangsmaterialien auf kostengünstige Weise und in wenigen Verfahrensschritten die Herstellung entsprechender Materialien ermöglicht.

Eine weitere Aufgabe der vorliegenden Erfindung steht in der Bereitstellung eines Verfahrens zur Herstellung poröser kohlenstoffbasierter Materialien, welches die Herstellung stabiler selbsttragender Strukturen bzw. Membranen oder Membranschichten aus porösem kohlenstoffbasiertem Material ermöglicht.

Die erfindungsgemäße Lösung der oben genannten Aufgaben besteht in einem Verfahren zur Herstellung von porösem, kohlenstoffbasiertem Material, welches die folgenden Schritte umfasst:
a) Bereitstellung einer Folie aus nicht faserigem Polymermaterial ausgewählt aus
   (i) Homo- oder Copolymeren von aliphatischen oder aromatischen Polyolefinen wie Polyethylen, Polypropylen, Polybuten, Polyisobuten, Polypenten; Polybutadien, Polyvinyle wie Polyvinylchlorid oder Polyvinylalkohol, Poly(meth)acrylsäure, Polyacrylnitril, Polyurethan, Polystyrol, Polytetrafluorethylen; Kollagen; Albumin, Gelatine, Hyaluronsäure, Stärke, Methylcellulose, Hydroxypropyl-cellulose, Hydroxypropylmethylcellulose, Carboxymethylcellulose-Phtalat, Nitrocellulose-beschichtetes Celluloseacetat; Polyvinylidenchlorid-beschichtetes Celluloseacetat; Wachs, Paraffinwachs, Fischer-Tropsch-Wachs; Kasein, Dextran, Polysacchariden, Fibrinogen, Poly(D,L-Lactide), Poly(D,L-Lactide-Co-Glycolide), Polyglycolide, Polyhydroxybutylate, Polyalkylcarbonate, Polyorthoester, Polyhydroxyvalerinsäure, Polydioxanone, Polyethylenterephtalat, Polymalatsäure, Polytartronsäure, Polyanhydriden, Polyphosphazenen, Polyaminosäuren; Polyethylenvinylacetat, Silikonen; Poly(Ester-Urethane), Poly(Ether-Urethane), Poly(Ester-Harnstoffe), Polyethylenoxid, Polypropylenoxid, Polytetramethylenglycol; Polyvinylpyrrolidon, Poly(vinyl-acetat-phatalat), sowie Mischungen von Homo-oder Copolymeren eines oder mehrerer der oben genannten
   (ii) Alkydharz, Chlorkautschuk, Epoxidharz, Formaldehydharz, (Meth)Acrylatharz, Phenolharz, Alkylphenolharz, Aminharz, Melaminharz, Cellulosenitrat Kolophonium, Vinylesterharz, Teer, Teerpech, Bitumen, Schellack, oder Kombinationen der genannten.
b) Karbonisierung der Polymerfolie in einer Atmosphäre, die im wesentlichen frei von Sauerstoff ist, bei Temperaturen im Bereich von 80°C bis 3500°C.

In bevorzugten Ausführungsformen der vorliegenden Erfindung wird die Karbonisierung der Polymerfolie in einer Atmosphäre, die im wesentlichen frei von Sauerstoff ist, bei Temperaturen im Bereich von 200°C bis 2500°C durchgeführt.

Erfindungsgemäß wurden gefunden, dass sich aus Polymerfolien aus geeigneten Polymermaterialien durch Karbonisierung bei hohen Temperaturen Kohlenstoffmaterialien erzeugen lassen, deren Porosität in Abhängigkeit vom verwendeten Polymerfilmmaterial, dessen Dicke und Struktur in weiten Bereichen gezielt einstellen lassen

Polymerfolien haben den Vorteil, dass sie einfach in nahezu beliebigen Dimensionen herstellbar sind oder kommerziell erhältlich sind. Polymerfolien sind leicht verfügbar und kostengünstig. Im Gegensatz zu Papier als Ausgangsmaterial für die Karbonisierung haben Polymerfolien den Vorteil, dass auch hydrophobe Materialien, die sich üblicherweise nicht mit den bei der Papierherstellung verwendeten Pulpen oder wasserverträglichen Naturfasern verwendeten Materialien, für die Herstellung kohlenstoffbasierter Materialien nutzen lassen.

Polymerfolien sind leicht formbar und können beispielsweise vor der Karbonisierung zu größeren Ensembles und Strukturen verarbeitet werden, wobei derartige Strukturen während der Karbonisierung des Polymerfolien materials im wesentlichen erhalten bleiben. Auf diese Art und Weise ist es möglich, durch vielfaches Aufeinanderschichten von Polymerfolien zu Folienpaketen und anschließende Karbonisierung gemäß dem Verfahren der vorliegenden Erfindung Paket- bzw. Modulstrukturen aus porösem kohlenstoffbasiertem Material zu erzeugen, welche aufgrund der mechanischen Festigkeit des resultierenden Materials als selbsttragende, mechanisch stabile Membran- bzw. Adsorberpakete in der Fluidtrennung vielfältig eingesetzt werden können.

Polymerfolien können vor der Pyrolyse und/oder Karbonisierung durch Falten, Prägen, Stanzen, Drucken, Extrudieren, Spritzen, Spritzgießen, Raffen und dergleichen in geeigneter Weise strukturiert werden und ggf. miteinander verklebt werden. Hierzu können übliche bekannte Klebstoffe und sonstige geeignete Klebematerialien wie z.B. Wasserglas, Stärke, Acrylate, Cyanacrylate, Heißschmelzklebstoffe, Kautschuk, oder lösemittelhaltige wie auch lösemittelfreie Klebstoffe etc.verwendet werden, wodurch das erfindungsgemäße Verfahren die Herstellung spezifisch konstruierter dreidimensionaler Strukturen mit geordnetem Aufbau aus dem erwünschten porösen kohlenstoffbasiertem Material ermöglicht.

Hierbei muss nicht zuerst das kohlenstoffbasierte Material hergestellt werden und dann im Nachhinein in aufwändigen Formgebungsschritten die gewünschte dreidimensionale Struktur, die für beispielsweise Membranpakete etc. erforderlich ist, hergestellt werden, sondern das erfindungsgemäße Verfahren ermöglicht es, die fertige Struktur des kohlenstoffbasierten Materials bereits vor der Karbonisierung durch geeignete Strukturierung bzw. Formung des Polymerfilms vorzugeben.

Somit können nach dem erfindungsgemäßen Verfahren auch diffizile kleinräumige Strukturen geschaffen werden, die aus fertigem Kohlenstoffinaterial im Wege der nachträglichen Formgebung nicht oder nur schwer zu bewerkstelligen sind. Hierbei kann z.B. der bei der Karbonisierung üblicherweise auftretende Schrumpf gezielt genutzt werden.

Die erfindungsgemäß verwendbaren Polymerfolien können flächenförmig in Blatt oder Bahnenform, z.B. als Rollenware, oder auch in Rohrform bzw. einer Röhren oder Kapillargeometrie bereitgestellt werden. Polymerfolien oder Kapillaren lassen sich z.B. mittels Phaseninversionsverfahren (asymmetrischer Schichtaufbau) aus Polymeremulsionen oder -Suspensionen herstellen.

Geeignete Polymerfolien im Verfahren der vorliegenden Erfindung sind beispielsweise Folien, Rohre oder Kapillaren aus Kunststoffen. Bevorzugte Kunststoffe bestehen aus Homo- oder Copolymeren von aliphatischen oder aromatischen Polyolefinen, wie Polyethylen, Polypropylen, Polybuten, Polyisobuten, Polypenten; Polybutadien; Polyvinyle wie Polyvinylchlorid oder Polyvinylalkohol, Poly(meth)-acrylsäure, Polyacrylnitril, Polyacrylcyanoacrylat; Polyurethan, Polystyrol, Polytetrafluorethylen; Kollagen, Albumin, Gelatine, Hyaluronsäure, Stärke, Methylcellulose, Hydroxypropyl-cellulose, Hydroxypropylmethylcellulose, Carboxymethylcellulose-Phtalat Celluloseacetat; Wachse, Paraffinwachse, Fischer-Tropsch-Wachse; Kasein, Dextrane, Polysaccharide, Fibrinogen, Poly(D,L-Lactide), Poly(D,L-Lactide-Co-Glycolide), Polyglycolide, Polyhydroxybutylate, Polyalkylcarbonate, Polyorthoester, Polyhydroxyvalerinsäure, Polydioxanone, Polyethylenterephtalat, Polymalatsäure, Polytartronsäure, Polyanhydride, Polyphosphazene, Polyaminosäuren; Polyethylenvinylacetat, Silikone; Poly(Ester-Urethane), Poly(Ether-Urethane), Poly(Ester-Harnstoffe), Polyethylenoxid, Polypropylenoxid, Pluronics, Polytetramethylenglycol; Polyvinylpyrrolidon, Poly(vinyl-acetat-phatalat), Mischungen von Homo- oder Copolymeren eines oder mehrerer der oben genannten.

Weitere Arten von Polymerfolien sind Polymerschaumsysteme, beispielsweise Phenolschäume, Polyolefinschäume, Polystyrolschäume, Polyurethanschäume, Fluoropolymerschäume, welche sich in einem nachfolgenden Karbonisierungsschritt in poröse Kohlenstoffmaterialien umwandeln lassen. Diese haben den Vorteil, dass sich im Karbonisierungsschritt Materialien mit in Abhängigkeit von der Schaumporosität einstellbarer Porenstruktur erzielen lassen. Zur Herstellung der geschäumten Polymere können alle üblichen Schäumungsverfahren des Standes der Technik unter Verwendung üblicher Treibmittel wie Halogenkohlenwasserstoffe, Kohlendioxid, Stickstoff, Wasserstoff und niedrig siedende Kohlenwasserstoffe verwendet werden. Auch können Füllstoffe in oder auf die Polymerfolien aufgebracht werden, welche geeignet sind eine Schaumbildung in oder auf der Polymerfolien zu bewirken.

Darüber hinaus können Polymerfolien auch nach Transferverfahren erhalten werden, wobei Materialien, Lacke, Überzüge, Laminate aus den oben genannten Materialien oder Polymermaterialien wie oben erwähnt auf Transfer-Trägermaterialien wie beispielsweise Folien aufgebracht werden, ggf. ausgehärtet werden und nachfolgend vom Trägermaterial abgelöst werden um anschliessend der Karbonisierung zugeführt zu werden.

Die Beschichtung des Trägermaterials kann hierbei durch geeignete Druckverfahren wie z.B. Rasterwalzendruck, Rakeln, Sprühverfahren, oder thermische, druckgepresste oder naß-in-naß Kaschierungen und dergleichen erfolgen. Mehrere dünne Schichten sind möglich und ggf. erwünscht, um z.B. Fehlerfreiheit des Polymerfilms zu gewährleisten. Ferner können bei der Aufbringung der Beschichtungen auf das Transfer-Trägermaterial ggf. verschiedene Rasterungen für eine möglichst homogene Lackverteilung verwendet werden.

Mit Transferverfahren der beschriebenen Art ist es auch möglich, Multilayer-Gradientenfilme aus verschiedenen Schichtmaterialabfolgen zu erzeugen, die nach der Karbonisierung kohlenstoffbasierte Gradientenwerkstoffe ergeben, in welchen beispielsweise die Dichte des Materials ortsabhängig variieren kann.

Sofern sehr dünne Polymerfilme zur Verwendung im erfindungsgemäßen Verfahren erforderlich sind, können diese nach dem Transferverfahren durch z.B. Pulverbeschichtung oder Schmelzbeschichtung auf geeigneten Folien erzeugt und dann abgelöst und karbonisiert werden. Sofern die Trägerfolie unter Karbonisierungsbedingungen vollständig zu verflüchtigen ist, wie z.B. bei Polyolefinfolien, ist eine Ablösung von der Trägerfolie ggf nicht notwendig oder sogar bevorzugt.

Beim Transferverfahren kann erfindungsgemäß die Polymerfolie aus einem Lackfilm sein, der aus Alkydharz, Chlorkautschuk, Epoxidharz, Formaldehydharz, (Meth)Acrylatharz, Phenolharz, Alkylphenolharz, Aminharz, Melaminharz, Cellulosenitrat Kolophonium, Novolac^{®} - Epoxidharze, Vinylesterharz, Teer, Teerpech, Bitumen, sowie Stärke, Schellack, Wachse, oder Kombinationen der genannten, erzeugt wurde.

Besonders bevorzugt sind Lacke auf Basis von Phenol- und/oder Melaminharzen, die gegebenenfalls ganz oder teilweise epoxidiert sein können, z. B. handelsübliche Emballagelacke, sowie Ein- oder Zwei-Komponentenlacke auf Basis gegebenenfalls epoxidierter aromatischer Kohlenwasserstoffharze.

Die verwendete Polymerfolie im Verfahren der vorliegenden Erfindung kann in bestimmten bevorzugten Ausführungsformen vor der Karbonisierung mit organischen und/oder anorganischen Verbindungen beschichtet imprägniert oder modifiziert werden. Eine ein- oder beidseitig aufgebrachte Beschichtung des Polymerfilms kann beispielsweise umfassen: Epoxidharze, Phenolharz, Teer, Teerpech, Bitumen, Kautschuk, Polychloropren oder Poly(styrol-co-butadien)-Latexmaterialien, Siloxane, Silikate, Metallsalze bzw. Metallsalzlösungen, beispielsweise Übergangsmetallsalze, Russ, Fullerene, Aktivkohlepulver, Kohlenstoffmolekularsieb, Perowskit, Aluminiumoxide, Siliziumoxide, Siliziumcarbid, Bornitrid, Siliziumnitrid, Edelmetallpulver wie beispielsweise Pt, Pd, Au oder Ag; sowie Kombinationen davon.

Bevorzugte Modifizierungen lassen sich z.B. durch oberflächliche Parylenierung oder Imprägnierung der Polymerfolien oder der daraus gewonnenen kohlenstoffbasierten Materialien gewinnen. Hierbei werden die Polymerfolien zunächst bei erhöhter Temperatur, üblicherweise etwa 600 °C mit Paracyclophan behandelt, wobei auf den Polymerfolien oder daraus erzeugten Materialien oberflächlich eine Schicht aus Poly(p-xylylen) ausgebildet wird. Dieser läßt sich ggf. in einem nachfolgenden Karbonisierungsschritt in Kohlenstoffumwandeln.

In besonders bevorzugten Ausführungsformen wird die Schrittfolge Parylenierung und Karbonisierung mehrfach wiederholt.

Durch ein- oder beidseitige Beschichtung des Polymerfilms mit oben genannten Materialien oder auch durch gezielten Einbau derartiger Materialien in die Polymerfilmstruktur lassen sich die Eigenschaften des nach der Karbonisierung resultierenden porösen kohlenstoffbasierten Materials gezielt beeinflussen und veredeln. Beispielsweise kann durch Einbau von Schichtsilikaten in den Polymerfilm oder Beschichtung des Polymerfilms mit Schichtsilikaten, Nanopartikeln, anorganischen Nanokompositen Metallen, Metalloxiden und dergleichen der thermische Ausdehnungskoeffizient des resultierenden Kohlenstoffmaterials wie auch dessen mechanische Eigenschaften oder Porositätseigenschaften modifiziert werden.

Die Aufbringung oder der Einbau von Metallen und Metallsalzen, insbesondere auch von Edelmetallen und Übergangsmetallen ermöglicht es, die chemischen und adsorptiven Eigenschaften des resultierenden porösen kohlenstoffbasierten Materials den jeweils erwünschten Erfordernissen anzupassen, so dass das resultierende Material für besondere Anwendungen beispielsweise auch mit heterogenkatalytischen Eigenschaften ausgerüstet werden kann.

In bevorzugten Ausführungsformen des erfindungsgemäßen Verfahrens werden die physikalischen und chemischen Eigenschaften des porösen kohlenstoffbasierten Materials nach der Pyrolyse bzw. Karbonisierung durch geeignete Nachbehandlungsschritte weiter modifiziert und dem jeweils gewünschten Verwendungszweck angepasst.

Geeignete Nachbehandlungen sind beispielsweise reduzierende oder oxidative Nachbehandlungsschritte, bei welchem das Material mit geeigneten Reduktionsmitteln und/oder Oxidationsmitteln wie Wasserstoff, Kohlendioxid, Wasserdampf, Sauerstoff, Luft, Salpetersäure und dergleichen sowie ggf. Mischungen dieser behandelt wird.

Die Nachbehandlungsschritte können ggf. bei erhöhter Temperatur, jedoch unterhalb der Pyrolysetemperatur, beispielsweise von 40°C bis 1000°C, vorzugsweise 70°C bis 900°C, besonders bevorzugt 100°C bis 850°C, insbesondere bevorzugt 200°C bis 800°C und insbesondere bei etwa 700 °C durchgeführt werden. In besonders bevorzugten Ausführungsformen wird das erfindungsgemäß hergestellte Material reduktiv oder oxidativ, oder mit einer Kombination dieser Nachbehandlungsschritte bei Raumtemperatur modifiziert.

Durch oxidative bzw. reduktive Behandlung, oder auch den Einbau von Zusatzstoffen, Füllstoffen oder funktionellen Materialien lassen sich die Oberflächeneigenschaften der erfindungsgemäß hergestellten Materialien gezielt beeinflussen bzw. verändern. Beispielsweise können durch Einbau von anorganischen Nanopartikeln oder Nanokompositen wie Schichtsilikaten die Oberflächeneigenschaften des Materials hydrophilisiert oder hydrophobisiert werden.

Weitere geeignete Zusatzstoffe, Füllstoffe oder funktionelle Materialien sind beispielsweise Silizium- oder Aluminiumoxide, Aluminosilikate, Zirkonoxide, Talkum, Graphit, Russ, Zeolithe, Tonmaterialien, Phyllosilikate, die dem Fachmann üblicherweise bekannt sind.

In bevorzugten Ausführungsformen kann die Einstellung der Porosität durch Herauswaschen von Füllstoffen wie beispielsweise Polyvinylpyrrolidon, Polyethylenglycol, Aluminiumpulver, Fettsäuren, Mikrowachse- oder -Emulsionen, Paraffine, Carbonate, gelöste Gase, oder wasserlösliche Salze mit Wasser, Lösemittel, Säuren oder Laugen oder durch Destillation oder oxidative bzw. nichtoxidative thermische Zersetzung erfolgen. Ggf. kann die Porosität auch durch Stukturierung der Oberfläche mit pulverförmigen Substanzen wie beispielsweise Metallpulver, Ruß, Phenolharzpulver erzeugt werden.

Der Zusatz von Aluminium-basierten Füllstoffen führt z.B. zu einer Erhöhung des thermischen Ausdehnungskoeffizienten und durch den Zusatz von Glas- , Graphit-oder Quartz-basierten Füllstoffen zu einer Verminderung des thermischen Ausdehnungskoeffizienten, so dass durch Mischung der Komponenten im Polymersystem der thermische Ausdehnungskoeffizient der erfindungsgemäßen Materialien entsprechend individuell eingestellt werden kann.

Auch können die erfindungsgemäß hergestellten Materialien nachträglich durch Einbau geeigneter Zusatzstoffe mit biokompatiblen Oberflächen ausgestattet und gegebenenfalls als Bioreaktoren oder Arzneistoffträger eingesetzt werden. Hierzu können z.B. Medikamente oder Enzyme in das Material eingebracht werden, wobei erstere ggf. durch geeignete Retardierung und/oder selektive Permeationseigenschaften der Membranen kontrolliert freigesetzt werden.

Ferner ist es in bestimmten Ausführungsformen bevorzugt, die erfindungsgemäß hergestellten Materialien zu fluorieren. In Abhängigkeit vom angewendeten Fluorierungsgrad können die erfindungsgemäßen Materialien bei hohem Fluorierungsgrad mit lipophoben Eigenschaften, bei geringem Fluorierungsgrad mit lipophilen Eigenschaften ausgestattet werden.

Darüber hinaus ist es ggf. bevorzugt, die erfindungsgemäßen Materialien durch Behandeln mit wasserlöslichen Substanzen wie z. B. Polyvinylpyrrolidon oder Polyethylenglykolen, Polypropylenglykolen zumindest oberflächlich zu hydrophilisieren.

Durch diese Maßnahmen lässt sich das Benetzungsverhalten der Materialien in gewünschter Weise modifizieren.

Das karbonisierte Material kann gegebenenfalls auch in einem weiteren optionalen Verfahrensschritt einem sogenannten CVD-Prozeß (Chemical Vapour Deposition, chemische Gasphasenabscheidung) unterzogen werden, um die Oberflächen oder Porenstruktur und deren Eigenschaften weiter zu modifizieren. Hierzu wird das karbonisierte Material mit geeigneten Precursorgasen bei hohen Temperaturen behandelt. Derartige Verfahren sind im Stand der Technik seit langem bekannt.

Als Kohlenstoff abspaltende Precursor kommen nahezu alle bekannten gesättigten und ungesättigten Kohlenwasserstoffe mit ausreichender Flüchtigkeit unter CVD-Bedingungen in Frage. Beispiele hierfür sind Methan, Ethan, Ethylen, Acetylen, lineare und verzweigte Alkane, Alkene und Alkine mit Kohlenstoffzahlen von C₁ - C₂₀, aromatische Kohlenwasserstoffe wie Benzol, Naphthalin, sowie ein- und mehrfach alkyl-, alkenyl- und alkinylsubstituierte Aromaten wie beispielsweise Toluol, Xylol, Cresol, Styrol etc.

Als Keramik-Precursor können BCl₃, NH₃, Silane wie Tetraethoxysilan (TEOS), SiH₄, Dichlorodimethylsilan (DDS), Methyltrichlorosilan (MTS), Trichlorosilyldichloroboran (TDADB), Hexadichloromethylsilyloxid (HDMSO), AlCl₃, TiCl₃ oder Mischungen davon verwendet werden.

Diese Precursor werden in CVD-Verfahren zumeist in geringer Konzentration von etwa 0,5 bis 15 Vol.-% in Mischung mit einem Inertgas, wie beispielweise Stickstoff, Argon angewendet. Auch der Zusatz von Wasserstoff zu entsprechenden Abscheidegasgemischen ist möglich. Bei Temperaturen zwischen 200 und 2000°C, vorzugsweise 500 bis 1500°C und besonders bevorzugt 700 bis 1300°C, spalten die genannten Verbindungen Kohlenwasserstofffragmente bzw. Kohlenstoff oder keramische Vorstufen ab, die sich im Porensystem des pyrolysierten Materials im wesentlichen gleichmäßig verteilt niederschlagen, dort die Porenstruktur modifizieren und so zu einer im wesentlichen homogenen Porengröße und Porenverteilung im Sinne einer weiteren Optimierung führen.

Zur Steuerung der gleichmäßigen Verteilung der abgeschiedenen Kohlenstoff- oder Keramikpartikel im Porensystem des karbonisierten Materials kann beispielsweise während der Abscheidung der Kohlenstoff-Precursor an einer Oberfläche des karbonisierten Gegenstands zusätzlich ein Druckgradient, z.B. in Form eines kontinuierlichen Unterdrucks bzw. Vakuums angelegt werden, wodurch die abgeschiedenen Partikel gleichmäßig in das Porengefüge des karbonisierten Stoffes eingesaugt werden (sogennante "forced flow CVI", Chemical Vapour Infiltration; siehe z.B. W. Benzinger et. all., Carbon 1996, 34, Seite 1465). Die so erzielte Homogenisierung des Porengefüges erhöht zudem die mechanische Festigkeit der so hergestellten Materialien.

Dieses Verfahren kann analog auch mit Keramik-, Sintermetall-, Metall- oder Metalllegierungs-Precursoren wie oben erwähnt angewendet werden.

Ferner können mittels Ionenimplantierung die Oberflächeneigenschaften des erfindungsgemäßen Materials modifiziert werden. So können durch Implantierung von Stickstoff Nitrid-, Carbonitrid- oder Oxynitridphasen mit eingelagerten Übergangsmetallen gebildet werden, was die chemische Resistenz und mechanische Widerstandsfähigkeit der kohlenstoffhaltigen Materialien deutlich erhöht. Die Ionenimplantierung von Kohlenstoff kann zur Erhöhung der mechanischen Festigkeit der erfindungsgemäßen Materialien wie auch zur Nachverdichtung poröser Materialien verwendet werden.

In weiters bevorzugten Ausführungsformen wird das erfindungsgemäß hergestellte Material nach der Pyrolyse und/oder Karbonisierung mit geeigneten Verfahren mechanisch zerkleinert, beispielsweise durch Mahlen in Kugel- oder Walzenmühlen und dergleichen. Das so hergestellte zerkleinerte Kohlenstoffmaterial kann als Pulver, Plättchen, Stäbe, Kugeln, Hohlkugeln verschiedener Körnungen verwendet werden, oder mittels üblicher Verfahren des Standes der Technik zu Granulaten oder Extrudaten verschiedenster Form verarbeitet werden. Auch Heißpressverfahren, ggf. unter Zusatz geeigneter Bindemittel können angewendet werden um das erfindungsgemäße Material zu formen. Besonders geeignet sind hierzu alle Polymere, die intrinsisch über Membraneigenschaften verfügen oder entsprechend hergestellt werden, um die o.g. Werkstoffe einzuarbeiten.

In den besonders bevorzugten Ausführungsformen des Verfahrens der vorliegenden Erfindung werden die Polymerfolien jedoch vor der Karbonisierung geeignet strukturiert, beispielsweise geprägt, zu Baugruppen miteinander verbunden, verklebt oder mechanisch miteinander verbunden, da sich hierdurch die Möglichkeit ergibt, auf einfache Weise leicht zu formendes Polymerfilmmaterial geeignet vorzustrukturieren, wobei die Struktur während des Karbonisierungsschrittes im wesentlichen erhalten bleibt.

Der Pyrolyse- bzw- Karbonisierungsschritt des erfindungsgemäßen Verfahrens wird üblicherweise bei Temperaturen im Bereich von 80°C bis 3500°C durchgeführt, vorzugsweise bei 200°C bis 2500°C, besonders bevorzugt bei 200°C bis 1200°C . Bevorzugte Temperaturen in einigen Ausführungsformen liegen bei 250°C bis 500°C. Die Temperatur wird vorzugsweise je nach den Eigenschaften der verwendeten Materialien so gewählt, dass die Polymerfolie mit möglichst geringem Temperaturaufwand im wesentlichen vollständig zu kohlenstoffhaltigem Feststoff überführt wird. Durch geeignete Wahl bzw. Steuerung der Pyrolysetemperatur kann die Porosität, die Festigkeit und Steife des Materials und weitere Eigenschaften eingestellt werden.

Die Atmosphäre beim Pyrolyse- bzw. Karbonisierungsschritt ist im erfindungsgemäßen Verfahren im wesentlichen frei von Sauerstoff. Bevorzugt ist die Verwendung von Inertgasatmosphären, beispielsweise aus Stickstoff, Edelgas wie Argon, Neon, sowie alle anderen inerten, nicht mit Kohlenstoff reagierenden Gase oder Gasverbindungen, reaktive Gase wie Kohlendioxid, Salzsäure, Ammoniak, Wasserstoff und Mischungen von inerten Gasen. Bevorzugt sind Stickstoff und/oder Argon. In einigen Fällen kann eine Aktivierung nach der Karbonisierung mit den reaktiven Gasen, zu denen dann auch Sauerstoff oder Wasserdampf gehört, erfolgen, um gewünschte Eigenschaften zu erzielen.

Die Karbonisierung im erfindungsgemäßen Verfahren wird üblicherweise bei Normaldruck in Gegenwart von inerten Gasen wie oben genannt durchgeführt. Gegebenenfalls sind jedoch auch höhere Inertgasdrücke vorteilhaft verwendbar. In bestimmten Ausführungsformen des erfindungsgemäßen Verfahrens kann die Karbonisierung auch bei Unterdruck bzw. im Vakuum erfolgen.

Der Karbonisierungsschritt wird vorzugsweise in einem kontinuierlichen Ofenprozess durchgeführt. Die ggf. strukturierten, beschichteten oder vorbehandelten Polymerfolien werden dabei auf einer Seite dem Ofen zugeführt und am anderen Ende des Ofens wieder austreten. In bevorzugten Ausführungsformen kann die Polymerfolie bzw. der aus Polymerfolien geformte Gegenstand im Ofen auf einer Lochplatte, einem Sieb oder dergleichen aufliegen, so dass durch den Polymerfilm während der Karbonisierung Unterdruck angelegt werden kann. Dies ermöglicht nicht nur eine einfache Fixierung der Gegenstände im Ofen, sondern auch eine Absaugung und optimale Durchströmung der Folien bzw. Baugruppen mit Inertgas während der Karbonisierung.

Der Ofen kann durch entsprechende Inertgasschleusen in einzelne Segmente unterteilt werden, in welchen nacheinander ein oder mehrere Karbonisierungsschritte, ggf. bei unterschiedlichen Karbonisierungsbedingungen wie zum Beispiel unterschiedlichen Temperaturstufen, unterschiedlichen Inertgasen bzw. Vakuum durchgeführt werden können.

Ferner können in entsprechenden Segmenten des Ofens ggf. auch Nachbehandlungsschritte wie Nachaktivieren durch Reduktion oder Oxidation oder Imprägnierung mit Metalle, Metallsalzlösungen oder Katalysatoren durchgeführt werden.

Alternativ hierzu kann die Pyrolyse/Karbonisierung auch in einem geschlossenen Ofen durchgeführt werden, was insbesondere dann bevorzugt ist, wenn die Karbonisierung im Vakuum durchgeführt werden soll.

Während der Karbonisierung im erfindungsgemäßen Verfahren tritt üblicherweise eine Gewichtsabnahme der Polymerfolie von ca. 5 % bis 95 %, vorzugsweise ca. 40 % bis 90 %, insbesondere 50 % bis 70 %, je nach verwendetem Ausgangsmaterial und Vorbehandlung auf. Darüber hinaus tritt während der Karbonisierung im erfindungsgemäßen Verfahren in der Regel ein Schrumpf der Polymerfolie bzw. der aus Polymerfolien erzeugten Struktur bzw. Baugruppe auf. Der Schrumpf kann in einer Größenordnung von 0 % bis etwa 95 %, vorzugsweise bei 10 % bis 30 % liegen.

Die erfindungsgemäß hergestellten Materialien sind chemisch stabil, mechanisch belastbar, elektrisch leitend und wärmebeständig.

Im erfindungsgemäßen Verfahren kann die elektrische Leitfähigkeit in Abhängigkeit von der verwendeten Karbonisierungstemperatur und der Art und Menge des eingesetzten Zusatzstoffs bzw. Füllmaterials in weiten Berreichen eingestellt werden. So kann bei Temperaturen im Bereich von 1000 bis 3500 °C infolge der auftretenden Graphitisierung des Materials eine höhere Leitfähigkeit erreicht werden als bei tieferen Temperaturen. Daneben kann die elektrische Leitfähigkeit aber auch beispielsweise durch Zusatz von Graphit zur Polymerfolie erhöht werden, welche dann bei niedrigeren Temperaturen karbonisiert werden kann.

Die erfindungsgemäß hergestellten Materialien weisen beim Erhitzen in inerter Atmosphäre von 20°C auf 600°C und anschließendem Abkühlen auf 20°C eine dimensionale Veränderung von maximal +/- 10 %, vorzugsweise maximal +/- 1 %, insbesondere bevorzugt maximal +/- 0,3 % auf.

Das erfindungsgemäß hergestellte poröse kohlenstoffbasierte Material weist, je nach Ausgangsmaterial, Menge und Art der Füllmaterialien, einen Kohlenstoffgehalt von mindestens 1 Gew.-% auf, vorzugsweise mindestens 25 %, gegebenenfalls auch mindestens 60 % und insbesondere bevorzugt mindestens 75 %. Erfindungsgemäß besonders bevorzugtes Material weist einen Kohlenstoffgehalt von mindestens 50 Gew.-% auf.

Die spezifische Oberfläche nach BET erfindungsgemäß hergestellte Materialien ist üblicheiweise sehr gering, da die Porosität kleiner ist als mit dieser Methode nachweisbar. Durch entsprechende Zusatzstoffe oder Verfahren (Porositätsgeber oder Aktivierung) lassen sich jedoch BET Oberflächen von über2000 m²/g erzielen.

Das nach dem erfindungsgemäßen Verfahren hergestellte Material in Blatt- oder Pulverform kann zur Herstellung von Membranen, Adsorbentien und/oder Membranmodulen bzw. Membranpaketen verwendet werden. Die Herstellung von Membranmodulen nach dem erfindungsgemäßen Verfahren kann beispielsweise wie in der WO 02/32558 beschrieben erfolgen, wobei anstelle der dort beschriebenen Papiergrundmatrix eine Polymerfolie verwendet wird.

Beispiele für die Verwendung des erfindungsgemäß hergestellten Materials im Bereich der Fluidtrennung sind: Allgemeine Gastrennung wie beispielsweise Sauerstoff-Stickstoffirennung zur Anreicherung von O₂ aus Luft, Trennung von Kohlenwasserstoffgemischen, Abtrennung von Wasserstoffen aus wasserstoffhaltigen Gasgemischen, Gasfiltration, Abtrennung von CO₂ aus Raumluft, Abtrennung flüchtiger organischer Verbindungen aus Abgasen oder Raumluft, Reinigung, Entsalzung, Enthärtung oder Gewinnung von Trinkwasser, als Brennstoffzellenelektrode, in Form von Sulzerpackungen, Raschigringen und dergleichen.

In einer besonderen Ausführungsform der vorliegenden Erfindung wird die Polymerfolie vor der Pyrolyse bzw. Karbonisierung auf übliche Adsorbermaterialien oder Membranen wie Aktivkohle, Zeolith, Keramik, Sintermetalle, Metalle oder Metalllegierungen und dergleichen, vorzugsweise auf Adsorbermaterialien in Form von Pellets oder Granulat aufgebrach.

Nach der Karbonisierung lassen sich so Adsorbermaterialien mit einer oberflächlichen Membranschicht herstellen, wodurch die Selektivität der Adsorber von der Selektivität der Membran bestimmt wird. Auf diese Weise lassen sich z.B. Adsorbergranulate herstellen, die selektiv nur die Stoffe adsorbieren können, die durch die Membran permeieren können. Eine rasche Erschöpfung des Adsorbers infolge Belegung mit unerwünschten Nebenkomponenten wird dadurch hinausgezögert bzw. vermieden. Hierdurch lassen sich die Austauschintervalle von Adsorberpatronen in entsprechenden Anwendungen verlängern, was zu einer erhöhten Wirtschaftlichkeit führt.

Bevorzugte Anwendungen derartiger Membran-beschichteter Adsorber liegen z.B. in PSA-Anlagen, in Fahr- oder Flugzeugkabinen, Atemschutzsystemen wie Gasmasken.

### Beispiele

Beispiel 1: Pyrolyse und Karbonisierung von beidseitig dünn mit Nitrozellulose beschichteter Zelluloseacetatfolie, Hersteller UCB Films, Typ Cellophane^{®} MS 500, Gesamtdicke 34.7 micron, 50g/m².

Die Folie wurden bei 830°C in gereinigter Stickstoffatmosphäre (Fließgeschwindigkeit 10 Liter/Min.) über eine Dauer von 48 Stunden in einem handelsüblichen Hochtemperaturofen pyrolysiert bzw. karbonisiert. Anschließend wurde der dabei auftretende Schrumpf durch Vergleich der gemittelten Meßwerte von jeweils drei rechteckigen Folienstücken und der daraus hergestellten Kohlenstoffblätter bestimmt. Die Ergebnisse sind in Tabelle 1 zusammengestellt.

**Tabelle 1: Schrumpf der Nitrozellulose-beschichteten Folie**

| Cellophane^{®} MS 500 | Vor Pyrolyse | Nach Pyrolyse | Differenz [%] |
|---|---|---|---|
| Länge a [mm] | 120 | 70 | 41,7 |
| Länge b [mm] | 60 | 44 | 26,7 |
| Fläche [mm²] | 7200 | 3080 | 57,2 |
| Gewicht [g] | 0,369 | 0,075 | 79,7 |

Anschließend wurden die Stickstoff- und die Wasserstoffpermeabilität der oben hergestellten Kohlenstoffblätter bei verschiedenen Bedingungen getestet. Die Bedingungen und Ergebnisse sind unten in Tabelle 2 aufgeführt. Die Permeabilitätswerte sind Mittelwerte aus jeweils drei Messungen.

**Tabelle 2: Membrandaten:**

| Gas | Temperatur | Druck | Zeit | Membranfläche | Permeabilität |
|---|---|---|---|---|---|
| | [°C] | [bar] | [sec] | [m²] | [l/m²*h*bar) |
| N₂ | 25 | 0,10 | Nicht messbar | 0,000798 | - |
| N₂ | 25 | 0,20 | Nicht messbar | 0,000798 | - |
| N₂ | 25 | 0,50 | Nicht messbar | 0,000798 | - |
| N₂ | 25 | 1,00 | Nicht messbar | 0,000798 | - |
| H₂ | 25 | 0,20 | 69,0 | 0,000798 | 33 |
| H₂ | 25 | 0,30 | 60,0 | 0,000798 | 25 |
| H₂ | 25 | 0,40 | 58,0 | 0,000798 | 19 |
| H₂ | 25 | 0,50 | 58,0 | 0,000798 | 16 |
| H₂ | 25 | 0,99 | 39,1 | 0,000798 | 12 |
| H₂ | 25 | 2,00 | 24,9 | 0,000798 | 9 |
| H₂ | 25 | 2,5 | Gerissen | 0,000798 | - |

Beispiel 2: Pyrolyse und Karbonisierung von beidseitig dünn mit Polyvinylidenchlorid (PVdC) beschichteten Zelluloseacetatfolien, Hersteller UCB Films, Typ Cellophane^{®} XS 500, Gesamtdicke 34.7 micron, 50g/m².

Die Folie wurden bei 830°C in gereinigter Stickstoffatmosphäre (Fließgeschwindigkeit 10 Liter/Min.) über eine Dauer von 48 Stunden in einem handelsüblichen Hochtemperaturofen pyrolysiert bzw. karbonisiert. Anschließend wurde der dabei auftretende Schrumpf durch Vergleich der gemittelten Meßwerte von jeweils drei rechteckigen Folienstücken und der daraus hergestellten Kohlenstoffblätter bestimmt. Die Ergebnisse sind in Tabelle 3 zusammengestellt.

**Tabelle 3: Schrumpf der PVdC-beschichteten Folie**

| Cellophane^{®} XS 500 | Vor Pyrolyse | Nach Pyrolyse | Differenz [%] |
|---|---|---|---|
| Länge a [mm] | 120 | 67 | 44,2 |
| Länge b [mm] | 60 | 41 | 31,7 |
| Fläche [mm²] | 7200 | 2747 | 61,9 |
| Gewicht [g] | 0,377 | 0,076 | 79,8 |

Beispiel 3: Pyrolyse und Karbonisierung von homogenen und fehlstellenfreien Epoxydharzfihnen, Gesamtdicke 7 micron vor Karbonisierung, 2,3 micron nach Karbonisierung.

Die Folie wurde durch ein Lösemittelverdampfungsverfahren aus einer 20 Gew.% Lösung hergestellt.
Die Karbonisierung erfolgte bei 830°C in gereinigter Stickstoffatmosphäre (Fließgeschwindigkeit 10 Liter/Min.) über eine Dauer von 48 Stunden in einem handelsüblichen Hochtemperaturofen. Anschließend wurde der dabei auftretende Schrumpf durch Vergleich der gemittelten Meßwerte von jeweils drei rechteckigen Folienstücken und der daraus hergestellten Kohlenstoffblätter bestimmt. Die Ergebnisse sind in Tabelle 4 zusammengestellt.

**Tabelle 4: Schrumpf der Epoxy-Folie**

| | Vor Pyrolyse | Nach Pyrolyse | Differenz [%] |
|---|---|---|---|
| Länge a [mm] | 100 | 46 | 54 |
| Länge b [mm] | 100 | 44 | 56 |
| Fläche [mm²] | 10.000 | 2024 | 78 |
| Gewicht [g] | 0.0783 | 0,0235 | 70 |

Das so hergestellte Blattmaterial wurde
a) in einem zweiten Aktivierungsschritt einer zweiten Temperaturbehandlung an Luft bei 350°C über 2 Stunden ausgesetzt.
b) In einem zweiten Schritt mit einer Kohlenwasserstoff-CVD-Schicht, durchgeführt bei 700°C in einer zweiten Temperaturbehandlung versehen.

Dadurch veränderte sich das Wasseraufnahmevermögen, das wie folgt gemessen wurde: 1ml VE-Wasser wurde mit einer Pipette auf die Folienoberfläche aufgesetzt (jeweils 20mm Durchmesser) und für 5 Minuten einwirken gelassen. Anschließend wurde die Gewichtsdifferenz ermittelt.

| | Wasseraufnahme [g] |
|---|---|
| Karbonisierte Probe | 0,0031 |
| a) Aktivierte Probe | 0,0072 |
| b) CVD-modifizierte Probe | 0,0026. |

Hieraus wird ersichtlich dass die CVD-Modifizierung die Porosität reduziert, während die Aktivierung die Porosität des Blattmaterials vergrößert.

Beispiel 4: Pyrolyse und Karbonisierung von homogenen und fehlstellenfreien Epoxydharzfilmen, Gesamtdicke 3g/m².

Die Folie wurde durch ein Lösemittelverdampfungsverfahren aus einer 15 Gew.% Epoxy-Beschichtungslösung, die mit 50% eines Polyethylenglykols (bezogen auf Epoxydlack, Mw 1.000g/mol) versetzt war im Tauchbeschichtungsverfahren auf Edelstahlsubstraten mit 25mm Durchmesser hergestellt.
Die Karbonisierung erfolgte bei 500°C in gereinigter Stickstoffatmosphäre (Fließgeschwindigkeit 10 Liter/Min.) über eine Dauer von 8 Stunden in einem handelsüblichen Hochtemperaturofen.
Anschließend wurde die Beschichtung bei 60°C über 30 Minuten im Ultraschallbad in Wasser ausgewaschen und gewogen.
Gewicht Ronde ohne Beschichtung: 1,2046g
Gewicht nach Beschichtung 1,2066g
Gewicht nach Karbonisierung 1,2061g
Gewicht nach Auswaschvorgang 1,2054g.

Durch den Auswaschvorgang kann die Porosität der Folien vergrößert werden.

## Patentansprüche

1. Verfahren zur Herstellung von porösem kohlenstoffbasiertem Material zur Fluidtrennung, umfassend die folgenden Schritte:
a) Bereitstellung einer Folie aus nicht faserigem Polymermaterial ausgewählt aus
(i) Homo- oder Copolymeren von aliphatischen oder aromatischen Polyolefinen wie Polyethylen, Polypropylen, Polybuten, Polyisobuten, Polypenten; Polybutadien, Polyvinyle wie Polyvinylchlorid oder Polyvinylalkohol, Poly(meth)acrylsäure, Polyacrylnitril, Polyurethan, Polystyrol, Polytetrafluorethylen; Kollagen, Albumin, Gelatine, Hyaluronsäure, Stärke, Methylcellulose, Hydroxypropyl-cellulose, Hydroxypropylmethylcellulose, Carboxymethylcellulose-Phtalat, Nitrocellulose-beschichtetes Celluloseacetat; Polyvinylidenchlorid-beschichtetes Celluloseacetat; Wachs, Paraffinwachs, Fischer-Tropsch-Wachs; Kasein, Dextran, Polysacchariden, Fibrinogen, Poly(D,L-Lactide), Poly(D,L-Lactide-Co-Glycolide), Polyglycolide, Polyhydroxybutylate, Polyalkylcarbonate, Polyorthoester, Polyhydroxyvalerinsäure, Polydioxanone, Polyethylentcrephtalat, Polymalatsäure, Polytartronsäure, Polyanhydriden, Polyphosphazenen, Polyaminosäuren; Polyethylenvinylacetat, Silikonen; Poly(Ester-Urethane), Poly(Ether-Urethane), Poly(Ester-Harnstoffe), Polyethylenoxid, Polypropylenoxid, Polytetramethylenglycol; Polyvinylpyrrolidon, Poly(vinyl-acetat-phatalat), sowie Mischungen von Homo-oder Copolymeren eines oder mehrerer der oben genannten;
(ii) Alkydharz, Chlorkautschuk, Epoxidharz, Formaldehydharz, (Meth)Acrylatharz, Phenolharz, Alkylphenolharz, Aminharz, Melaminharz, Cellulosenitrat Kolophonium, Vinylesterharz, Teer, Teerpech, Bitumen, Schellack, oder Kombinationen der genannten.
b) Karbonisierung der Polymerfolie in einer Atmosphäre, die im wesentlichen frei von Sauerstoff ist, bei Temperaturen im Bereich von 80°C bis 3500°C.

2. Verfahren nach Anspruch 1,
**dadurch gekennzeichnet, dass** die Polymerfolie flächenförmig, in Blatt- oder Bahnenform, oder in Rohrform vorliegt.

3. Verfahren nach einem der Ansprüche 1 oder 2,
**dadurch gekennzeichnet, dass** die Polymerfolie vor der Karbonisierung durch Prägen, Falten, Stanzen, Drucken, Extrudieren, oder Kombinationen dieser Maßnahmen strukturiert wird.

4. Verfahren nach einem der vorhergehenden Ansprüche,
**dadurch gekennzeichnet, dass** die Polymerfolie Zusatzstoffe oder Füllstoffe enthält.

5. Verfahren nach Anspruch 4,
**dadurch gekennzeichnet, dass** die Zusatz- oder Füllstoffe aus Silizium- oder Aluminiumoxiden, Aluminosilikaten, Zirkonoxiden, Talkum, Graphit, Russ, Zeolithen, Tonmaterialien, Phyllosilikate, Wachs, Paraffin, Salze, Metalle, Metallverbindungen, oder aus löslichen organischen Verbindungen wie Polyvinylpyrrolidon oder Polyethylenglycol ausgewählt sind.

6. Verfahren nach Anspruch 4 oder 5,
**dadurch gekennzeichnet dass** die Füllstoffe durch Herauswaschen mit Wasser, Lösemittel, Säuren, oder Laugen, oder durch oxidatives oder nicht-oxidatives thermisches Zersetzen aus der Matrix entfernt werden.

7. Verfahren nach einem der Ansprüche 4 bis 6,
**dadurch gekennzeichnet, dass** die Füllstoffe in Form von Pulvern vorliegen.

8. Verfahren nach einem der Ansprüche 4 bis 7,
**dadurch gekennzeichnet, dass** die Füllstoffe geeignet sind, eine Schaumbildung in oder auf dem Polymerfilm zu bewirken.

9. Verfahren nach einem der vorhergehenden Ansprüche,
**dadurch gekennzeichnet, dass** das Material im Anschluss an die Karbonisierung einer oxidativen und/oder reduktiven Nachbehandlung unterzogen wird.

10. Verfahren nach einem der vorhergehenden Ansprüche,
**dadurch gekennzeichnet, dass** mehrere Polymerfolien zu einem Folienpaket aufeinandergeschichtet und gegebenenfalls miteinander verklebt und nachfolgend karbonisiert werden.

## Claims

1. A method for the preparation of porous carbon-based material for the separation of fluids, comprising the following steps:
a) provision of a foil (film) of non-fibrous polymer material selected from
i) homo- or copolymers of aliphatic or aromatic polyolefins such as polyethylene, polypropylene, polybutene, polyisobutene, polypentene, polybutadiene, polyvinyls such as polyvinyl chloride or polyvinyl alcohol, poly(meth)acrylic acid, polyacrylonitrile, polyurethane, polystyrene, polytetrafluorethylene; collagen, albumin, gelatin, hyaluronic acid, starch, methylcellulose, hydroxypropylcellulose, hydroxypropylmethylcellulose carboxymethylcellulose phthalate, nitrocellulose coated cellulose acetate, polyvinylidene chloride coated cellulose acetate, wax, paraffin wax, Fischer-Tropsch wax, casein, dextran, polysaccharide, fibrinogen, poly(D,L-lactide), poly(D,L-lactide-co-gycolide), polyglycolide, polyhydroxybutylate, polyalkylcarbonate, polyorthoester, polyhydroxyvaleric acid, polydioxanone, polyethylenetherephthalate, polymalic acid, polytartronic acid, polyanhydrides, polyphosphazenes, polyamino acids, polyethylene vinyl acetate, silicones, poly(ester-urethanes), poly(ether urethanes), poly(ester urea), polyethylene oxide, polypropylene oxide, polytetramethylene glycol; polyvinylpyrrolidone, poly(vinyl acetate phthalate) as well as mixtures of one or more of these homo- or copolymers,
ii) alkyd resin, chlorinated rubber, epoxy resin, formaldehyde resin, (meth)acrylate resin, phenol resin, alkyl phenol resin, amine resin, melamine resin, cellulose nitrate, colophony, vinyl ester resin, tar, tar pitch, bitumen, shellac or combinations thereof.
b) carbonization of the polymer foil in an atmosphere that is essentially free of oxygen at temperatures in the range of 80 °C to 3,500 °C.

2. The method according to claim 1,
**characterized in that** the polymer foil is provided in laminar form, in sheet or web form or in tube form.

3. The method according to claim 1 or claim 2,
**characterized in that** the polymer foil is structured prior to carbonization by stamping, folding, die-cutting, printing, extruding or combinations thereof.

4. The method according to any of the previous claims,
**characterized in that** the polymer foil comprises additives or fillers.

5. The method according to claim 4,
**characterized in that** the additives or fillers are selected from silicon or aluminum oxides, aluminosilicates, zirconium oxides, talcum, graphite, carbon black, zeolites, clay materials, phyllosilicates, wax, paraffin, salts, metals, metal compounds, or from soluble organic compounds such as e.g. polyvinylpyrrolidone or polyethylene glycol.

6. The method according to claim 4 or claim 5,
**characterized in that** the fillers are removed from the matrix by washing out with water, solvent, acids, or bases, or by oxidative or non-oxidative thermal decomposition.

7. The method according to any of claims 4 to 6,
**characterized in that** the fillers are present in form of powders.

8. The method according to any of claims 4 to 7,
**characterized in that** the fillers are suitable to cause foam formation in or on the polymer foil.

9. The method according to any of the previous claims,
**characterized in that** the material is subjected to an oxidative and/or reducing aftertreatment subsequent to carbonization.

10. The method according to any of the previous claims,
**characterized in that** several polymer foils are layered on top of each other to form a film package and wherein said polymer foils are optionally adhesively bonded together and are subsequently carbonized.

## Revendications

1. Procédé de fabrication de matériau poreux à base de carbone pour la séparation de fluides, comprenant les étapes suivantes :
a) préparation d'une feuille en matériau polymère non fibreux, sélectionné parmi :
(i) des homo- ou copolymères de polyoléfines aliphatiques ou aromatiques, tels que polyéthylène, polypropylène, polybutène, polyisobutylène, polypentène ; polybutadiène, polyvinyles, tels que chlorure de polyvinyle ou alcool polyvinylique, acide polyméthacrylique, polyacrylonitrile, polyuréthanne, polystyrène, polytétrafluoroéthylène ; collagène, albumine, gélatine, acide hyaluronique, amidon, méthylcellulose, hydroxypropylcellulose, hydroxypropyl-méthylcellulose, phtalate de carboxyméthylcellulose, acétate de cellulose revêtu dé nitrocellulose, acétate de cellulose revêtu de chlorure de polyvinylidène ; cire, cire de paraffine, cire de Fischer-Tropsch ; caséine, dextrane, polysaccharides, fibrinogène, poly(D,L-lactide), poly(D,L-lactide-co-glycolide), polyglycolide, polyhydroxybutylate, polyalkylcarbonate, polyortho-ester, acide polyhydroxyvalérique, polydioxanone, polyéthylène-téraphtalate, acide polymalique, acide polytartronique, polyanhydrides, polyphosphazènes, acides polyaminés ; polyéthylènevinylacétate, silicones ; poly(ester-uréthanne), poly(éther-uréthanne), poly(ester-urées), oxyde de polyéthylène, oxyde de polypropylène, polytétra-méthylèneglycol ; polyvinylpyrrolidone, poly(vinyle-acétate-phatalate), ainsi que des mélanges d'homo- ou de copolymères de l'un ou plusieurs de ceux cités ci-dessus ;
(ii) résine alkyde, caoutchouc au chlore, résine époxy, résine formaldéhyde, résine méthacrylique, résine phénolique, résine alkylphénolique, résine amine, résine de mélamine, nitrate de cellulose, colophane, résine ester vinylique, goudron, brai, bitume, gomme-laque, ou des combinaisons de ceux cités,
b) carbonisation de la feuille polymère dans une atmosphère essentiellement exempte d'oxygène, à des températures dans la plage de 80°C à 3500°C.

2. Procédé suivant la revendication 1, **caractérisé en ce que** la feuille polymère est présente en forme de feuille ou de nappe planiforme, ou en forme tubulaire.

3. Procédé suivant l'une des revendications 1 ou 2, **caractérisé en ce que** la feuille polymère est structurée avant la carbonisation par matriçage, pliage, estampage, compression, extrusion, ou par combinaisons de ces mesures.

4. Procédé suivant l'une des revendications précédentes, **caractérisé en ce que** la feuille polymère renferme des additifs ou des charges.

5. Procédé suivant la revendication 4, **caractérisé en ce que** les additifs ou charges sont sélectionnés parmi des oxydes de silicium ou d'aluminium, des aluminosilicates, des oxydes de zirconium, du talc, du graphite, de la suie, des zéolithes, des matériaux argileux, des phyllosilicates, de la cire, de la paraffine, des sels, des métaux, des combinaisons métalliques, ou parmi des combinaisons organiques solubles telles que polyvinylpyrrolidone ou polyéthylèneglycol.

6. Procédé suivant l'une des revendications 4 ou 5, **caractérisé en ce que** les charges sont éliminées de la matrice par lavage à l'eau, aux solvants, aux acides ou bases, ou par décomposition thermique oxydative ou non-oxydative.

7. Procédé suivant l'une des revendications 4 à 6, **caractérisé en ce que** les charges sont présentes en forme de poudres.

8. Procédé suivant l'une des revendications 4 à 7, **caractérisé en ce que** les charges sont aptes à provoquer un moussage dans ou sur le film polymère.

9. Procédé suivant l'une des revendications précédentes, **caractérisé en ce que** le matériau est soumis, à la suite de la carbonisation, à un post-traitement d'oxydation et/ou de réduction.

10. Procédé suivant l'une des revendications précédentes, **caractérisé en ce que** plusieurs feuilles polymères sont stratifiées en un paquet de feuilles et éventuellement collées entre elles, puis carbonisées.
